**Europäisches Patentamt**

**European Patent Office**

**Office européen des brevets**

(19)

(11) Veröffentlichungsnummer : **0 370 329 B1**

(12) **EUROPÄISCHE PATENTSCHRIFT**

(45) Veröffentlichungstag der Patentschrift :
29.05.91 Patentblatt 91/22

(51) Int. Cl.$^5$ : **C07C 249/14**

(21) Anmeldenummer : **89120841.5**

(22) Anmeldetag : **10.11.89**

(54) Verfahren zur Verbesserung der Lagerfähigkeit von geschmolzenem Cyclohexanonoxim.

(30) Priorität : **23.11.88 DE 3839448**

(43) Veröffentlichungstag der Anmeldung :
**30.05.90 Patentblatt 90/22**

(45) Bekanntmachung des Hinweises auf die
Patenterteilung :
**29.05.91 Patentblatt 91/22**

(84) Benannte Vertragsstaaten :
**BE CH DE ES FR GB IT LI NL**

(56) Entgegenhaltungen :
**DE-A- 2 135 781**
**US-A- 3 941 838**

(73) Patentinhaber : **BASF Aktiengesellschaft**
**Carl-Bosch-Strasse 38**
**W-6700 Ludwigshafen (DE)**

(72) Erfinder : **Fuchs, Hugo, Dr.**
**Egellstrasse 28**
**W-6700 Ludwigshafen (DE)**
Erfinder : **Agar, David, Dr.**
**Eichhornshoehe 37 a**
**W-6149 Rimbach (DE)**
Erfinder : **Neubauer, Gerald, Dr.**
**Mozartstrasse 24**
**W-6940 Weinheim (DE)**
Erfinder : **Ritz, Josef, Dr.**
**Osloer Weg 8**
**W-6700 Ludwigshafen (DE)**

## Beschreibung

Bei der Herstellung von Cyclohexanonoxim durch Umsetzung von Cyclohexanon mit wäßriger Hydroxylammonium-Ammoniumsulfatlösung bei einer Temperatur über dem Schmelzpunkt von Cyclohexanonoxim ohne Mitverwendung von Lösungsmitteln oder Puffersalzen bei niederen pH-Werten, wie es in der US-PS 4 031 139 beschrieben wird, erhält man Cyclohexanonoxim, das neben geringen Mengen an nicht umgesetztem Cyclohexanon eine wäßrige Lösung von Ammoniumbisulfat mit einem pH-Wert < 2,0 enthält. Da es häufig nicht möglich ist die erzeugten Mengen an Cyclohexanonoxim in dem Maße weiterzuverarbeiten wie es anfällt ist es notwendig, dieses zwischenzulagern. Hierbei hat sich herausgestellt, daß mit Cyclohexanonoxim, das relativ kurze Zeit gelagert wurde, bei der nachfolgenden Herstellung von Caprolactam die erforderlichen Kennzahlen hinsichtlich UV-Zahl und Permanganat-Zahl nicht mehr erreicht werden.

Aus der US-PS 3 941 838 ist auch schon bekannt, daß man Cyclohexanonoxim das durch Oximierung von Cyclohexanon mit Hydroxylammonium-Salzlösungen unter Neutralisation mit Ammoniak erhalten wurde, anschließend mit konzentrierter Ammoniumsulfatlösung mit einem pH-Wert von 3 bis 6 oder Hydroxylammonium-Salzlösungen behandelt, um den Wassergehalt zu senken. Ein Hinweis wie nach einem anderen Verfahren hergestelltes Cyclohexanonoxim in seiner Lagerstabilität verbessert werden kann, wird nicht gegeben.

Es war deshalb die technische Aufgabe gestellt, die Lagerfähigkeit von geschmolzenem Cyclohexanonoxim, das durch sogenannte saure Oximierung erhalten worden ist, zu verbessern, die Bildung von Cyclohexenon während der Lagerung zu vermindern und die Schwierigkeiten bei der Verwendung von so gelagertem Cyclohexanonoxim bei der Herstellung von Caprolactam zu vermeiden.

Diese Aufgabe wird gelöst in einem Verfahren zur Verbesserung der Lagerfähigkeit von geschmolzenem Cyclohexanonoxim, das 0,5 bis 15 Gew.-% Cyclohexanon und 1 bis 8 Gew.-% wäßrige Ammoniumbisulfatlösung enthält, wobei man das geschmolzene Cyclohexanonoxim mit einer 10- bis 42 gew.-%igen wäßrigen Ammoniumsulfatlösung wäscht und einen pH-Wert von 4,5 bis 5,8 einhält.

Das neue Verfahren hat den Vorteil, daß Cyclohexanonoxim, das aus der sauren Oximierung erhalten wurde, mindestens einen Tag problemlos gelagert werden kann, ohne daß bei der Weiterverarbeitung zu Caprolactam nachteilige Folgen eintreten. Ferner hat das neue Verfahren den Vorteil, daß selbst bei einem höheren Gehalt an Cyclohexanon die Bildung von Cyclohexenon zurückgedrängt wird.

Als Ausgangsverbindung verwendet man geschmolzenes Cyclohexanonoxim, das 0,5 bis 15 Gew.-% insbesondere 0,5 bis 2 Gew.-% Cyclohexanon, sowie 1 bis 8, insbesondere 4 bis 8 Gew.-% wäßrige Ammoniumbisulfatlösung enthält. Die im geschmolzenen Cyclohexanonoxim enthaltene wäßrige Ammoniumbisulfatlösung hat in der Regel einen Gehalt von 0,01 bis 0,5 Gew.-% Ammoniumbisulfat und hat einen pH-Wert von < 2,0, z.B. von 0,5 bis 1,5. Eine geeignete Schmelze von Cyclohexanonoxim erhält man beispielsweise durch Umsetzen von Cyclohexanon und wäßriger Hydroxylammonium-Ammoniumsulfatlösung bei einer Temperatur oberhalb des Schmelzpunkts von Cyclohexanonoxim in Abwesenheit von Lösungsmitteln, Neutralisationsmitteln und/oder Puffersalzen, bis das Gleichgewicht erreicht ist. Zweckmäßig wird die Umsetzung im Gegenstrom durchgeführt. Ein geeignetes Verfahren wird beispielsweise beschrieben in der US-PS 4 031 139.

Das zu behandelnde Cyclohexanonoxim hat vorteilhaft eine Temperatur von 70 bis 95°C, insbesondere von 70 bis 85°C.

Erfindungsgemäß wird das geschmolzene Cyclohexanonoxim mit einer 10 bis 42 gew.-%igen wäßrigen Ammoniumsulfat gewaschen, wobei man einen pH-Wert von 4,5 bis 5,8 einhält. Besonders vorteilhaft hält man einen pH-Wert von 5,2 bis 5,8 ein. Es ist auch vorteilhaft, eine 15 bis 35 gew.-%ige wäßrige Ammoniumsulfatlösung zu verwenden.

Vorteilhaft wendet man je Volumenteil Cyclohexanonoxim 0,1 bis 10, insbesondere 0,5 bis 5 Volumenteile wäßrige Ammoniumsulfatlösungen der genannten Konzentration an.

Es ist möglich, das Auswaschen absatzweise in einem Rührkessel durchzuführen, wobei man durch Zugabe von wäßriger Ammoniaklösung den pH-Wert aufrecht erhält. Vorteilhaft wäscht man das zu behandelnde Cyclohexanonoxim mehrmals z.B. bis zu 5 mal. Vorteilhaft führt man das Auswaschen kontinuierlich durch, indem man in einer Kolonne unten geschmolzenes Cyclohexanonoxim zugibt und im Gegenstrom von oben wäßrige Ammoniumsulfatlösung zugibt. Um eine gute Durchmischung zu gewährleisten, eignen sich beispielsweise Drehscheibenkolonnen, Mixer-Settler-Vorrichtungen oder Füllkörperkolonnen. Vorteilhaft gibt man im Verlauf der Kolonne wäßrige Ammoniaklösungen zu in dem Maße wie es erforderlich ist, um den genannten pH-Wert aufrecht zu erhalten.

Cyclohexanonoxim eignet sich zur Herstellung von Caprolactam.

Das Verfahren nach der Erfindung sei an folgenden Beispielen veranschaulicht.

Beispiel 1

Cyclohexanonoxim mit einem Gehalt von 10 Gew.-% Cyclohexanon, welches im Gleichgewicht mit einer 20 gew.-%igen wäßrigen Ammonbisulfatlösung stand, wurde 5 mal mit der gleichen Volumenmenge einer 23 gew.-%igen wäßrigen Ammoniumsulfatlösung mit einem pH-Wert von 5,5 bei einer Temperatur von 80°C gewaschen. Die Färbung des so behandelten Cyclohexanonoxim und die Bildung von Verunreinigungen wurden über 75 Stunden bei 90°C verfolgt. Eine Verfärbung des behandelten Cyclohexanonoxim trat erst nach 24 Stunden ein. Nach 75 Stunden erreichte der Gehalt an Cyclohexen-2-on 30 ppm und die Konzentration an UV-aktiven Verunreinigungen insgesamt 230 ppm.

Vergleichsbeispiel 1

Unbehandeltes Cyclohexanonoxim, wie in Beispiel 1 verwendet, zeigt bereits nach 4 Stunden bei 90°C eine gelbliche Färbung, der Gehalt an UV-aktiven Verunreinigungen betrug 230 ppm und der Gehalt an Cyclohexen-2-on 30 ppm.

Beispiel 2

Man verfährt wie in Beispiel 1 beschrieben, verwendet jedoch 40 gew.-%ige wäßrige Ammoniumsulfalösung mit einem pH von 5,5. Eine Färbung des behandelten Oxims bei 90°C wurde erst nach 24 Stunden beobachtet. Nach 50 Stunden betrug die Konzentration von UV-aktiven Verbindungen 200 ppm und der Gehalt an Cyclohexen-2-on 21 ppm.

## Ansprüche

1. Verfahren zur Verbesserung der Lagerfähigkeit von geschmolzenem Cyclohexanonoxim, das 0,5 bis 15 Gew.-% Cyclohexanon und 1 bis 8 Gew.-% wäßrige Ammoniumbisulfatlösung enthält, dadurch gekennzeichnet, daß man das geschmolzene Cyclohexanonoxim mit einer 10 bis 42 gew.-%igen wäßrigen Ammoniumsulfatlösung wäscht und einen pH-Wert von 4,5 bis 5,8 einhält.

2. Verfahren nach Anspruch 1, dadurch gekennzeichnet, daß man den pH-Wert durch Zugabe von wäßriger Ammoniaklösung einstellt.

3. Verfahren nach den Ansprüchen 1 und 2, dadurch gekennzeichnet, daß man einen pH-Wert von 5,2 bis 5,8 einhält.

4. Verfahren nach den Ansprüchen 1 bis 3, dadurch gekennzeichnet, daß man 15 bis 35 gew.-%ige wäßrige Ammoniumsulfatlösung verwendet.

5. Verfahren nach den Ansprüchen 1 bis 4, dadurch gekennzeichnet, daß man eine Temperatur von 75 bis 90°C einhält.

6. Verfahren nach den Ansprüchen 1 bis 5, dadurch gekennzeichnet, daß man je Volumenteil Cyclohexanonoxim 0,1 bis 10 Volumen-Teile wäßrige Ammoniumsulfat-Lösung verwendet.

7. Verfahren nach den Ansprüchen 1 bis 6, dadurch gekennzeichnet, daß man Cyclohexanonoxim kontinuierlich am unteren Ende einer Waschkolonne zuführt und wäßrige Ammoniumsulfatlösung von oben nach unten durch die Kolonne leitet und am oberen Ende der Kolonne Cyclohexanonoxim und am unteren Ende wäßrige Ammoniumsulfatlösung entnimmt.

## Claims

1. A process for improving the storability of a molten cyclohexanone oxime containing from 0.5 to 15% by weight of cyclohexanone and from 1 to 8% by weight of aqueous ammonium bisulfate solution, which comprises washing the molten cyclohexanone oxime with a 10-42% strength by weight aqueous ammonium sulfate solution and maintaining a pH of 4.5-5.8.

2. A process as claimed in claim 1, wherein the pH is set by the addition of aqueous ammonia solution.

3. A process as claimed in claim 1 or 2, wherein a pH of 5.2-5.8 is maintained.

4. A process as claimed in any one of claims 1 to 3, wherein 15-35% strength by weight aqueous ammonium sulfate solution is used.

5. A process as claimed in any one of claims 1 to 4, wherein 75-90°C is maintained.

6. A process as claimed in any one of claims 1 to 5, wherein per part by volume of cyclohexanone oxime from 0.1 to 10 parts by volume of aqueous ammonium sulfate solution are used.

7. A process as claimed in any one of claims 1 to 6, wherein the cyclohexanone oxime is continuously introduced at the bottom end of a wash column, an aqueous ammonium sulfate solution is passed downward through the column, and cyclohexanone oxime is removed at the top end of the column and aqueous ammonium sulfate solution at the bottom end.

## Revendications

1. Procédé d'amélioration de l'aptitude au stockage de cyclohexanone-oxime à l'état fondu qui contient de 0,5 à 15% en poids de cyclohexanone et de 1 à 8% en poids de solution aqueuse de bisulfite d'ammonium, caractérisé en ce qu'on lave la cyclohexanone-oxime à l'état fondu avec une solution aqueuse à 10-42% en poids de sulfate d'ammonium et on maintient un pH de 4,5 à 5,8.

2. Procédé selon la revendication 1, caractérisé en ce qu'on règle le pH par addition de solution aqueuse d'ammoniac.

3. Procédé selon la revendication 1 ou 2, caractérisé en ce qu'on maintient un pH de 5,2 à 5,8.

4. Procédé selon l'une quelconque des revendications 1 à 3, caractérisé en ce qu'on utilise une solution aqueuse à 15-35% en poids de sulfate d'ammonium.

5. Procédé selon l'une quelconque des revendications 1 à 4, caractérisé en ce qu'on maintient une température de 75 à 90°C.

6. Procédé selon l'une quelconque des revendications 1 à 5, caractérisé en ce qu'on utilise, par partie en volume de cyclohexanone-oxime, de 0,1 à 10 parties en volume de solution aqueuse de sulfate d'ammonium.

7. Procédé selon l'une quelconque des revendications 1 à 6, caractérisé en ce qu'on introduit de façon continue la cyclohexanone-oxime à l'extrémité inférieure d'une colonne de lavage, on envoie la solution aqueuse de sulfate d'ammonium de haut en bas à travers la colonne et on recueille la cyclohexanone-oxide à l'extrémité supérieure de la colonne et la solution aqueuse de sulfate d'ammonium à son extrémité inférieure.